# EUROPEAN PATENT APPLICATION

(11) **EP 1 187 418 A2**
(43) Date of publication of application: **13.03.2002**
(21) Application number: 01119067.5
(22) Date of filing: 07.08.2001
(51) Int. Cl.: H04L 29/06, G06F 17/60

(54) **Image distibution apparatus**

(30) Priority: 10.08.2000 JP 2000243491
(71) Applicant: Nidek Co., Ltd., Gamagori-shi, Aichi 443-0035 (JP)
(72) Inventor: Ueda, Atsuhiro, Kawabe-gun,Hyogo-ken, 666-0251 (JP)
(74) Representative: Kuhnen & Wacker

(57) **Abstract**

An image distribution apparatus distributes moving images of a surgical spot picked up during a treatment such as a surgical operation to more than one client without requiring expert knowledge on computers and communication. The image distribution apparatus comprises dial-up communication means (30) for carrying out digital communication with one or more clients, Internet communication means (60) for carrying out digital communication with one or more clients, interface means (26) for at least receiving a moving image signal picked up by image pickup means attached to a medical device, an encoding server (20) which encodes the moving image signal with a digitized image signal at one or more preset data rates in real time, and image distribution means (40) for distributing at least one digitized image signal at one or more of the data rates to the one or more clients through the telephone network or the Internet.

## Description

### BACKGROUND OF THE INVENTION

### 1. Field of the Invention

The present invention relates to an image distribution apparatus distributing moving images to more than one client. More particularly, the present invention relates to an image distribution apparatus distributing moving images of an action of a patient and/or a medical doctor and the patient's condition, picked up during a medical treatment such as a surgical operation, to more than one computer at a remote location in real time.

### 2. Description of Related Art

Lately, real-time distribution of moving images is already on its way to become possible attributed to increases in speed of computer processing and improvements in communication capacity of the Internet. Conventionally, hardware and software for the real-time distribution of moving images have been sold separately, and those who intend to distribute images join them to make image distribution possible.

However, those who intend to distribute images have to be furnished with hardware and software necessary for image distribution, and they have to install all the software in the hardware as well as to set up details necessary for image distribution. Accordingly, great effort and expert knowledge on computers and digital communication are needed for actual implementation of image distribution. In addition, this kind of the image distribution system is for distributing images picked up by a regular video camera. A distribution system for distributing images picked up by an image pickup device attached to a medical optical device such as a surgical microscope has never been existed.

### SUMMARY OF THE INVENTION

The present invention has been made in view of the above circumstances and has an object to overcome the above problems and to provide an image distribution apparatus for distributing moving images of a surgical spot picked up during a medical treatment such as a surgical operation to more than one client without requiring expert knowledge on computers and communication.

Additional objects and advantages of the invention will be set forth in part in the description which follows and in part will be obvious from the description, or may be learned by practice of the invention. The objects and advantages of the invention may be realized and attained by means of the instrumentalities and combinations particularly pointed out in the appended claims.

To achieve the objects and in accordance with the purpose of the present invention, as embodied and broadly described herein, an image distribution apparatus of this invention comprises dial-up communication means for carrying out digital communication with one or more clients through a telephone network, Internet communication means for carrying out digital communication with one or more clients through the Internet, interface means for at least receiving a moving image signal picked up by image pickup means attached to a medical device, image encoding means for encoding the moving image signal to a digitized image signal at one or more preset data rates in real time, and image distribution means for distributing at least one digitized image signal at one or more of the data rates to the one or more clients through the telephone network or the Internet.

According to this structure of the image distribution apparatus, the image pickup device attached to the medical optical device and connected with the interface for image input is utilized for picking up images, whereby the moving images picked up by the image pickup device can be distributed in real-time to more than one client connected with the subject apparatus through the telephone network and to clients connected with the subject apparatus through the Internet when communication capacity of the connection between each client and the present apparatus is greater than one or more of the data rates of the digitized image signals generated by the encoding unit.

In this condition, the medical optical device is not particularly specified, but for example, the device may be a surgical microscope or a slit lamp.

Further, the apparatus of the present invention is preferably provided with voice input means for inputting a voice signal, voice grade selecting means for selecting a voice grade for encoding, voice encoding means for encoding the voice signal inputted by the voice input means to a digitized voice signal at a data rate conforming to the voice grade selected by the voice grade selecting means, and voice distribution means for distributing the digitized voice signal to the one or more clients through the telephone network or the Internet.

In this structure, the image distribution apparatus of the present invention is capable of distributing digitized voice signals, as well as the digitized image signals, to plural clients. In addition, because the voice grade selection means is provided, the data rate of digitized voice signals to be distributed can be changed to a desired value.

Moreover, it may be arranged that the interface means should receive plural moving image signals, that the image encoding means should encode each of the plural moving image signals to a digitized image signal, and that the image distribution means should distribute all or a part of the plural digitized image signals to the one or more clients. In this structure, plural moving images are distributed to the clients in remote locations.

Further, the image distribution means may distribute only an image signal selected from among the plural image signals in accordance with an image selecting signal from the client. In such a manner, the clients can choose the images to be distributed to them from plural images.

In addition, the image distribution apparatus preferably comprises data rate memory means for prestoring plural sets of data rate groups consisted of one or more of the data rates, and data rate setting means for setting the data rate of the digitized image signal by selecting from the plural stored sets of the data rate groups, and wherein the image encoding means encodes the image signal to the digitized image signal at all the rates included in the selected data rate group. In such a manner, the data rate of a digitized image signal generated by the encoding unit can be easily changed to a suitable value.

Also, the image distribution apparatus is preferably provided with interface means for receiving a measurement signal indicating a value of a vital sign including at least one selected from temperature, blood pressure, pulses, the number of breaths, and oxygen saturation in the blood, measurement signal encoding means for encoding the measurement signal to a digitized measurement signal in real time, and measurement signal distribution means for distributing the digitized measurement signal to the one or more clients through the telephone network or the Internet. In such a manner, the vital sign information can be simultaneously distributed along with moving images to remote locations in real time.

Further, the image distribution apparatus is preferably provided with a patient identifying data input means, connected with a patient information database, for inputting patent identifying data identifying a patient to be picked up by the image pickup means, and data distribution means for searching patient data corresponding to the patient identifying data through the patient information database when the patient identifying data are inputted by the patient identifying data input means, and for distributing all or a part of the patient data to the one or more clients through the telephone network or the Internet. In such a manner, when the patient identifying data corresponding to a patient to be operated are inputted, the patient data of the patient are searched through the patient information database, and the patient data are distributed along with moving images.

According to one aspect of the image distribution apparatus consistent with the present invention, what users of the subject apparatus have to do for image distribution is to connect the image pickup device attached to the medical optical device with the interface for image input and to commence picking up images. This provides a way to distribute the moving images in real time, which are picked up by the image pickup device from the same direction as an operator's sight during a medical treatment such as a surgical operation, to more than one client in remote locations. It means that the users are required neither to go through complex setting operations on the subject apparatus nor to have expert knowledge on computers and/or communication technology. Further, since each client can be connected with the subject apparatus through the telephone network, the clients who have no access to the Internet can also be connected with the apparatus.

According to another aspect of the present invention, in addition to digitized image signals, digitized voice signals can be distributed to plural clients. Further, since the voice grade selecting means is provided, it is possible to change the data rate of the digitized voice signals to be distributed to the desired values. Moreover, it is possible that relatively small communication capacity of the Internet or the telephone network is flexibly allotted for digitized image signals and digitized voice signals with reference to the speed of the movements of the subject to be picked up and the bandwidth of the voice to be recorded. Therefore, it is possible to distribute images and voice in real time as the loss of the information of both the images and voice is suppressed. For example, when human voice of which bandwidth is narrow is inputted, a large amount of the communication capacity is used for image distribution by setting the voice grade low. On the other hand, when the subject to be picked up displays few movements, voice with high audio quality can be distributed by setting the voice grade high.

Further, according to yet another aspect of the present invention, the image distribution apparatus is so configured as to allow plural moving images to be distributed to the clients in remote locations. According to yet another aspect of the present invention, the clients can choose the images to be distributed from plural images.

Moreover, according to yet another aspect of the present invention, it is possible to easily change the data rate of digitized image signals generated by the encoding unit to suitable values.

Also, according to yet another aspect of the present invention, the vital sign information can be distributed to remote locations along with the moving pictures, whereby it is possible to convey detailed information of the conditions during a surgical operation to recipients of image distribution in real time.

In addition, according to yet another aspect of the present invention, when the patient identifying data corresponding to a patient to be operated are inputted, the patient data of the patient are searched through the patient identifying database, and the patient data are distributed along with the moving images. This makes recipients of image distribution observe the moving images picked up during a surgical operation as they recognize the patient's background from a medical viewpoint.

### BRIEF DESCRIPTION OF THE DRAWINGS

The accompanying drawings, which are incorporated in and constitute a part of this specification, illustrate embodiments of the present invention and, together with the description, serve to explain the objects, advantages and principles of the invention. In the drawings,
Fig. 1 is a view showing a schematic configuration of an exemplary image distribution system as one preferred embodiment consistent with the present invention;
Fig. 2 is a block diagram showing a configuration of an image distribution apparatus 10;
Fig. 3 is a view showing a structure of distribution setting data tables of the image distribution apparatus 10;
Fig. 4 is a flowchart showing distribution setup processing carried out on a streaming server;
Fig. 5 is a flowchart showing encoding setup processing of setting an encoding server 20;
Fig. 6 is a flowchart showing setup processing for distributing data of a patient to be operated regarding image and voice distribution;
Fig. 7 is to describe the distribution processing and shows a flowchart showing distribution processing and screens displayed to a client;
Fig. 8 is view showing a layout of a screen displayed on a display unit of the client at the time of receiving image streaming signals from the streaming server; and
Fig. 9 is a view showing a layout of a distribution setting screen.

### DETAILED DESCRIPTION OF THE PREFERRED EMBODIMENTS

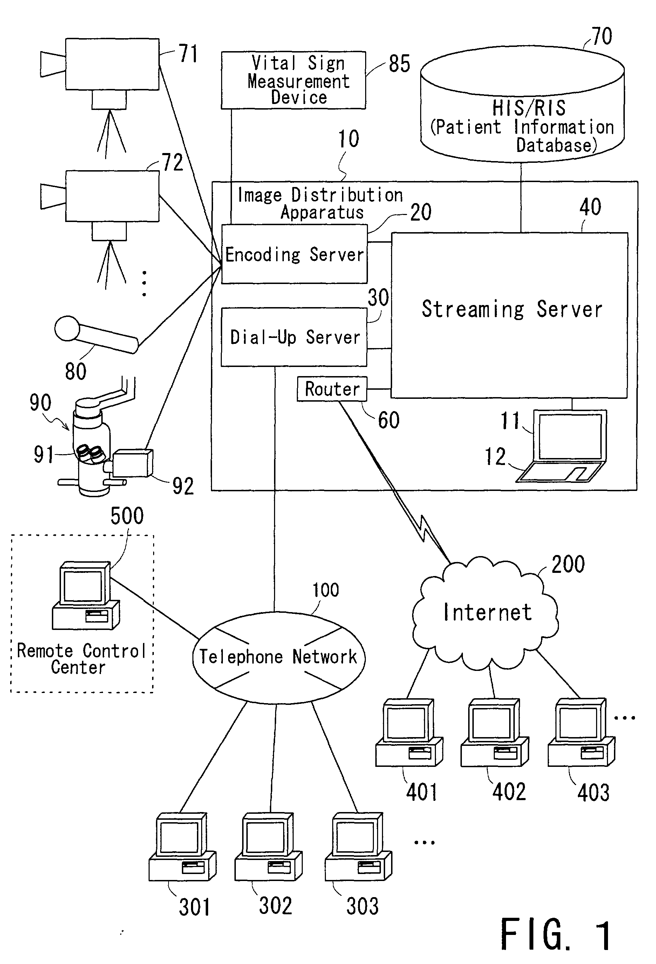

A detailed description of one preferred embodiment of an image distribution apparatus embodying the present invention will now be given referring to the accompanying drawings. Fig. 1 is a view showing a schematic configuration of an exemplary image distribution system as one preferred embodiment the present invention. Fig. 1 shows an image distribution apparatus 10 having a display unit 11 such as a CRT and an LCD, an input unit 12 such as a keyboard and a mouse, an encoding server 20, a dial-up server 30, a streaming server 40, and a router 60. The encoding server 20 is connected with video cameras 71, 72, ..., a microphone 80, a vital sign measurement device 85 measuring temperature, blood pressure, and pulses, and an image pickup device 92 attached to a medical optical device 90 such as a surgical microscope and a slit lamp (In this specification, a surgical microscope is used.) The encoding sever 20 is also connected with the streaming server 40.

The video cameras 71, 72, ... each has a CCD image pickup element and is provided with an oscillating mechanism for their up-and-down, side-to-side movements and a zooming mechanism driven by a motor. The oscillating mechanism and the zooming mechanism are controlled by control signals inputted from the streaming server 40 via the encoding server 20. Each of the video cameras 71, 72, ... is set at different positions from the others. For example, the video camera 71 is aimed at an entire operating room, and the video camera 72 is aimed at the hands of a surgical operator. Then, each of the video cameras 71, 72, ... picks up an image of a subject to be picked up and converts the image into electric signals to output to the encoding server 20.

The medical optical device 90 has light path dividing means, such as a half mirror, disposed on a light path between an objective lens and an eyepiece, and the image pickup device 92 is disposed on a light path divided therefrom. The image pickup device 92 having a CCD image pickup element is provided with a mechanism for changing aiming directions of an image pickup for the up-and-down, side-to-side movements and a zooming mechanism driven by a motor. Those mechanisms are controlled by control signals inputted from the streaming server 40 via the encoding server 20. The same image as the image observed by the operator through the medical optical device 90, or a zoom-in or zoom-out image of the same is converted into electric signals to be outputted to the encoding server 20. Also, the microphone 80 converts voice into electric signals to output to the encoding server 20.

The dial-up server 30 is connected with a telephone network 100 as well as with the streaming server 40. Plural clients (client computers) 301, 302, 303, ... are connected with the telephone network 100. Also, a remote control computer 500 installed at a remote control center is connected with the telephone network 100. The router 60 connecting the streaming server 40 with the Internet 200 controls the communication between them. Plural clients 401, 402, 403, ... are connected with the Internet 200. The router 60 may be connected with the Internet 200 by a dedicated line or by a telephone line. The remote control computer 500 may be connected with the router 60 by a dedicated line.

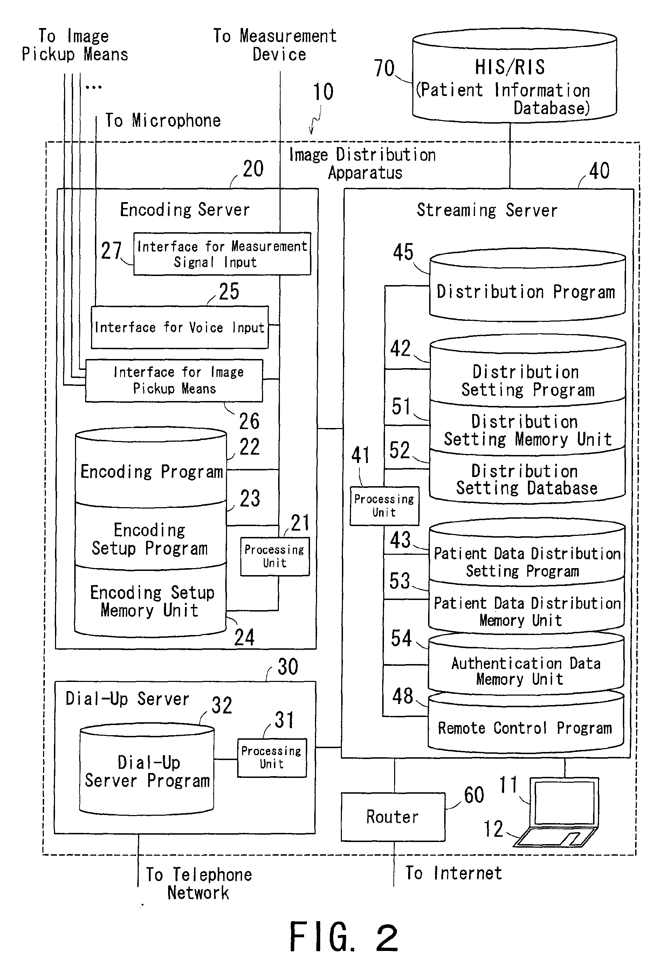

Next, a description of a detailed configuration of the image distribution apparatus 10 will be given. Fig. 2 is a block diagram showing the configuration of the image distribution apparatus 10. The encoding server 20 has a processing unit 21 constituted of a CPU (central processing unit), ROM, RAM, and others, an encoding program 22, an encoding setup program 23, an encoding setup memory unit 24, an interface for voice input 25 connected with the microphone 80, an interface for image pickup means 26 receiving image signals from the image pickup means, such as video cameras, and sending control signals thereto, and an interface for measurement signal input 27 connected with the vital sign measurement device 85. The processing unit 21 carries out processing based on the encoding program 22 and the encoding setup. The processing unit 21 encodes each signal given from the video cameras 71, 72, ..., the microphone 80, and the image pickup device 92 in accordance with the encoding program 22 and to give the streaming server 40 digitized image signals and digitized voice signals generated by encoding. Further, the processing unit 21 gives the streaming server 40 vital sign data based on signals, which are inputted from the vital sign measurement device 85 and indicate blood pressure, and so on.

The dial-up server 30 includes a processing unit 31 constituted of a CPU (central processing unit), ROM, RAM, and others, and a dial-up server program 32. The processing unit 31 performs in accordance with the dial-up server program 32. The dial-up server 30 is in charge of a connection for communication between the clients 301, 302, 303, ... through the telephone network 100 and the streaming server 40.

The streaming server 40 is provided with a processing unit 41 constituted of a CPU (central processing unit), ROM, RAM, and others, and the processing unit 41 performs based on programs and data stored in the streaming server 40. The streaming server 40 stores a distribution setting program 42, a patient data distribution setting program 43, a distribution program 45, a remote control program 48, a distribution setting memory unit 51, a distribution setting database 52, a patient data distribution memory unit 53, and an authentication data memory unit 54. Further, the streaming server 40 is connected with a patient information database 70, such as HIS (Hospital Information System)/RIS (Radiology Information System), storing patient chart data and/or X-ray image data.

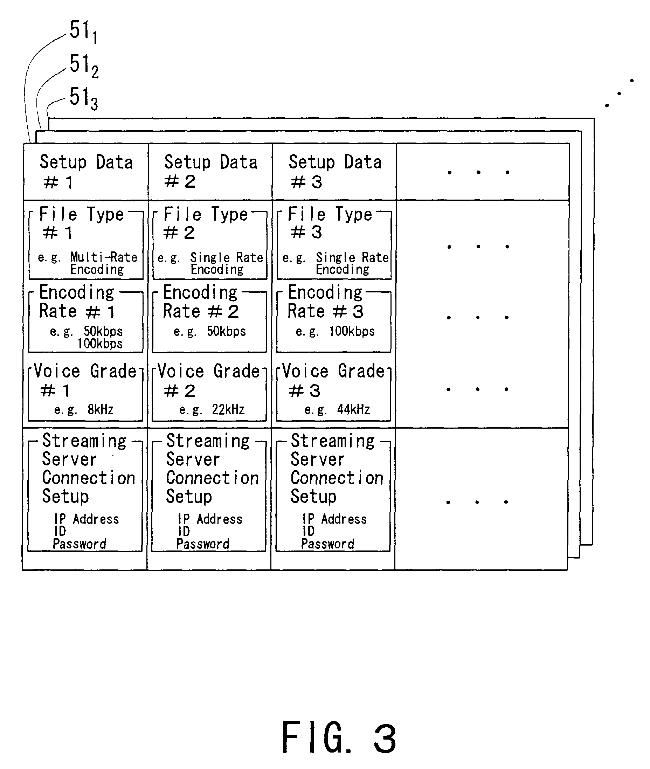

The distribution setting database 52 prestores distribution setting data tables 51₁, 51₂, 51₃, ... having the distribution setting data which are appropriate for distributing images and voice in accordance with each hospital department. Fig. 3 is a view showing a structure of the distribution setting data tables. The distribution setting data tables 51ₙ (n=1, 2, 3, ...) store more than one set of setting data, and each set is a combination of a file type, an encoding rate, a voice grade, and a method for connecting to the streaming server 40.

The file type indicates the choice between a single rate encoding and a multi-rate encoding. The single rate encoding is to encode each image signal to digitized image signals at a single rate, and the multi-rate encoding is to encode each image signal to digitized image signals at plural encoding rates. An encoding rate indicates a bit rate of a bit stream obtained by encoding an image. A voice grade indicates a bit rate of a bit stream obtained by encoding voice. The method for connecting the encoding server 20 with the streaming server 40 includes an IP address, an ID, and a password.

For example, according to the first setup data, the file type is set for the multi-rate encoding, the encode rates are set at 50 kbps and 100 kbps, and the voice grade is set at 8 kHz. According to the second setup data, the file type is set for the single rate encoding, the encoding rate is set at 50 kbps, and the voice grade is set at 22 kHz. According to the third setup data, the file type is set for the single rate encoding, the encoding rate is set at 100 kbps, and the voice grade is set at 44 kHz. It should be noted that all the setup data adapt the same setup method for setting up the connection to the streaming server 40.

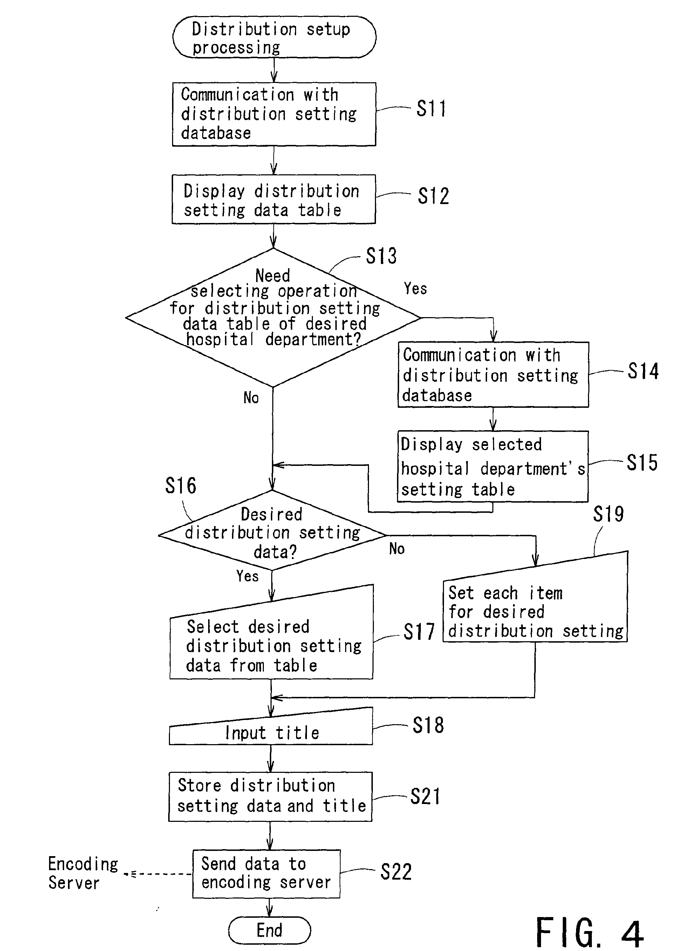

Next, Fig. 4 is a flowchart showing a distribution setup processing carried out on the streaming server 40. This distribution setup processing is carried out by the processing unit 41 in accordance with the distribution setting program 42 stored in the streaming sever 40. First, in a step S11, the processing unit 41 communicates with the distribution setting database 52, and in a step S12, the processing unit 41 displays one of the distribution setting data tables 51₁, 51₂, 51₃, ... on the display unit 11.

When the distribution setting data table of the desired hospital department is not displayed at this point, a user operates the input unit 12 for selecting the distribution setting data table of the desired hospital department (step S13 = "YES"), whereby the processing unit 41 communicates with the distribution setting database 52 (step S14), and the distribution setting data table of the desired hospital department is displayed on the display unit 11 (step S15). On the other hand, when the distribution setting data table of the desired hospital department is displayed, the processing goes on without carrying out the selecting operation for selecting the distribution setting data table of the desired hospital department (step S13 = "NO").

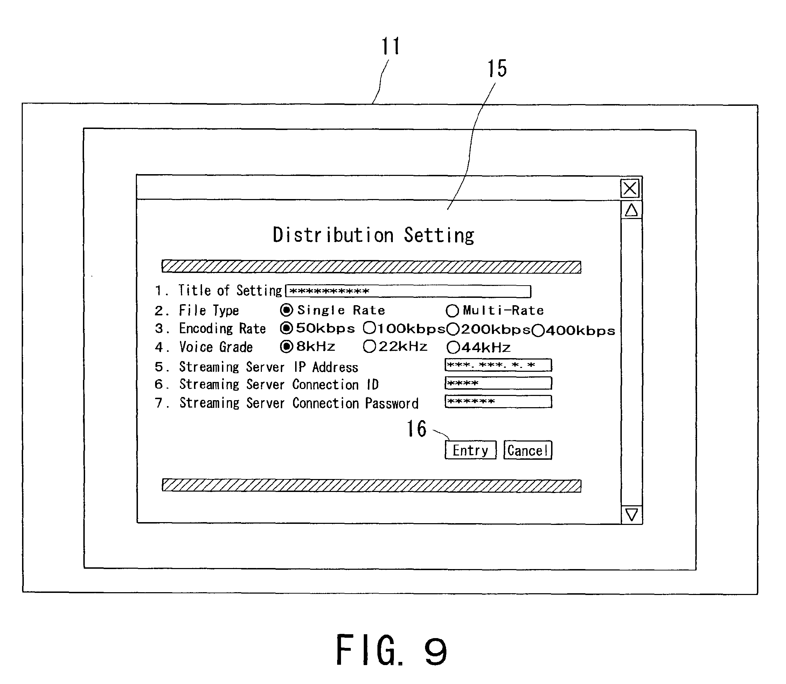

At this stage, when the desired distribution setting data do not exist on the distribution setting data tables 51₁, 51₂, 51₃, ... (step S16 = "NO"), a distribution setting display 15 for setting the details of distribution is displayed on the display unit 11 (step S19). Fig. 9 is a view showing a layout of the distribution setting screen 15. On this distribution setting screen 15, setup items including a title of the subject setting, a file type (single rate or multi-rate), an encoding rate (50 kbps, 100 kbps, 200 kbps, and 400 kbps), a voice grade (8 kHz, 22 kHz, and 44 kHz), an IP address, an ID number for connection, and a password for connection, can be selected to proceed to a step S18. When the multi-rate encoding is selected at this point, more then one encoding rate can be selected.

In such a manner, when the desired distribution setting data do not exist on the distribution setting data tables 51ₙ (n=1, 2, 3, ...), selecting the various items for the distribution setting may be selected on the distribution setting display 15, whereby efficient distribution is possible according to purposes. For instance, on the occasion of distributing picked-up images and recorded voice in a lecture and the like where fewer movements and more oral information are usually involved, selecting a low image encoding rate and a high voice grade makes it possible to distribute images with little missing information and voice with high audio quality as well as to suppress the load of the processing for encoding and distributing and the data rates of distribution (images and voice) at the time of distribution. On the occasion of distributing moving images during a surgical operation, selecting a high image encoding rate and a low voice grade makes it possible to distribute images with high image quality as well as to suppress the load of the processing for encoding and distributing and the data rates of distribution (images and voice) at the time of distribution.

Then, when an entry button 16 is clicked, the information of the setup items is stored in the distribution setting database 52, whereby the information of the setup items may be selected as one of the distribution setting data tables 51ₙ thereafter. Further, regarding the voice grade, quantized bit numbers (for example, 8 bits or 16 bits) may be selected for the three kinds of the above-mentioned frequencies to set up more details.

On the other hand, when the distribution setting data table of the desired hospital department is shown in the step S16 (step S16 = "YES"), the user may select the necessary setup data displayed on the distribution setting data table by using the input unit 12 for distributing the subject images and others (step S17). In such a manner, when the necessary distribution setting data are prestored on the distribution setting data tables 51ₙ (n=1, 2, 3, ...), it is easy for the user to carry out the distribution setting since he/she only needs to select a hospital department and a data set from the plural sets of the setup data for the distribution setting.

In a step S18, a title of distribution of the subject images and others is inputted. Then, the processing unit 41 processes the distribution setting memory unit 51 included in the streaming server 40 to store the information of the distribution setting and the inputted title (step S21) and sends them to the encoding server 20 (step S22).

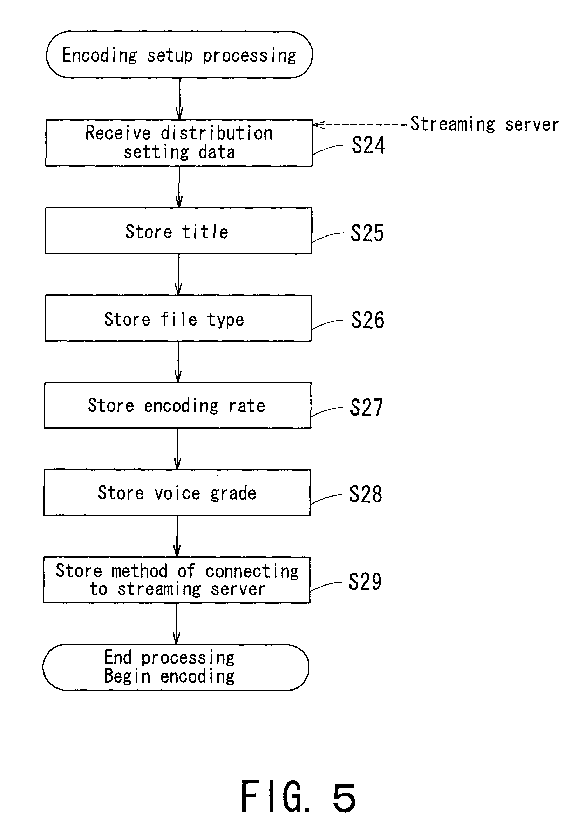

Fig. 5 is a flowchart showing an encoding setup processing of setting the encoding server 20. The processing unit 21 carries out this processing in accordance with the encoding setup program 23 stored in the encoding server 20. The encoding server 20 encodes image signals and voice signals based on this encoding setup and gives digitized streaming signals generated by encoding to the streaming server 40.

First, in a step S24, the distribution setting data given from the streaming server 40 in the step S19 are received. In accordance with these distribution setup data, the encoding setup memory unit 24 included in the encoding server 20 stores the title in a step S25, the file type in a step S26, the encoding rate in a step S27, the voice grade in a step S28, and the method for connecting to the streaming server 40 in a step S29. In such a manner, the user can complete the encoding setup of the encoding server 20 only by carrying out a distribution setting operation on the streaming server 40. In other words, it is possible for the user to select necessary setup values for encoding, such as encoding rates, without directly operating on the encoding server 20.

In accordance with the encoding program 22, the processing unit 21 encodes each of inputted image signals and voice signals based on the above-mentioned encoding setup processing for inputted image signals and voice signals, and the unit 21 sends them to the streaming server 40. For example, when the multi-rate encoding is selected for the file type, and when 50 kbps and 100 kbps are selected for the encoding rates, each image signal is encoded to a digitized bit streaming signal at the data rate of 100 kbps as well as at the data rate of 50 kbps. When the voice grade is set at 8 kHz, the voice signals inputted from the microphone 80 are encoded to produce a digitized bit stream at the data rate of the voice grade at 8 kHz. Then, the processing unit 21 gives the two sets of the image bit streaming signals and a voice bit stream to the streaming server 40 in accordance with the selected connecting method.

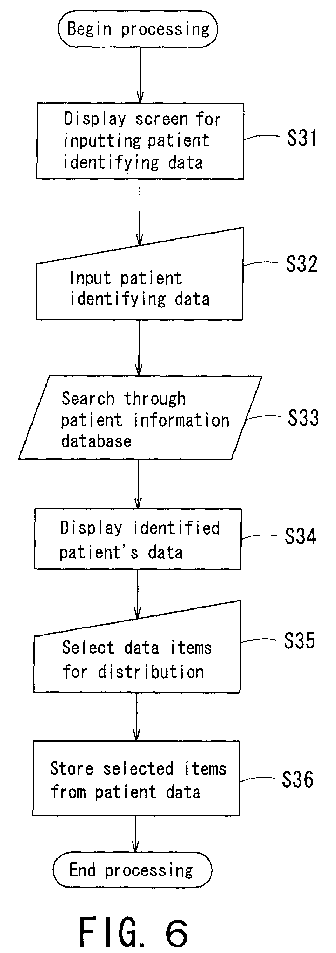

Next, Fig. 6 is a flowchart showing a setup processing for distributing data of a patient to be operated regarding image and voice distribution. The processing unit 41 curries out this setup processing in compliance with the patient data distribution setting program 43. First, the screen prompting for patient identifying data is shown on the display unit 11 (step S31). As the patient identifying data, a hospital ID card number, a patient chart number, a patient number, a patient's name, the patient's birth date, and so on, are inputted to identify a patient. When the user inputs the patient identifying data (step S32), the patient data, identified by the inputted data and including his/her name, address, age, birth date, gender, diagnosis, condition, treatment and medical histories, and X-ray image, are searched through the databases of HIS/RIS (step S33) to be displayed (step S34).

Then, the user selects the data items to be distributed along with images and voice from the patient data by using the input unit 12 (step S35). The data items selected in such a manner are stored in the patient data distribution memory unit 53 by the processing unit 41 (step S36). It is also possible for the user to select part of data items, instead of all the data items, from the selected items. It means that, for example, the user does not need to distribute the data items related to the patient's privacy such as his/her name and/or address, or the data items irrelevant to the surgical operation, by not selecting them for distribution. In another way, the data items that are related to the patient's privacy and should not be distributed may be predetermined, whereby the user is not allowed to select those preset data items for distribution in the step S35. In such a manner, the data items related to the patient's privacy can be prevented from being selected for distribution by mistake.

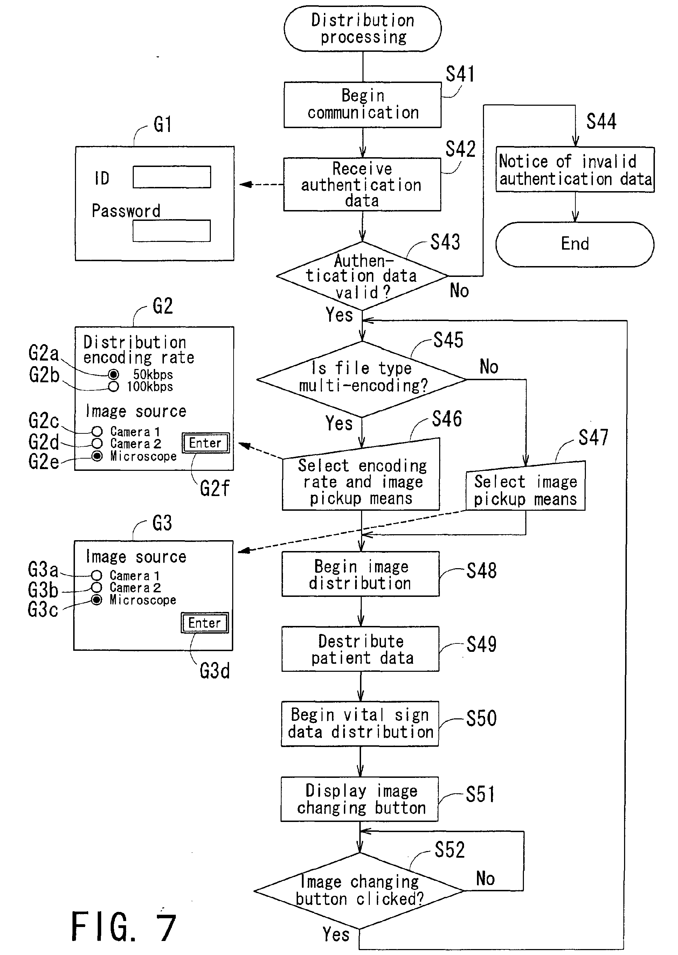

Next, Fig. 7 is to describe the distribution processing and shows a flowchart showing distribution processing and screens displayed to a client. The description of the processing of distributing images and others by the image distributing apparatus 10 will be given below in accordance with this flowchart. To carry out the processing, the processing unit 41 executes the distribution program 45 stored in the streaming server 40. While this image distribution apparatus 10 distributes images to plural clients 301, 302, 303, ... connected through the telephone network 100 and to plural clients 401, 402, 403, ... connected through the Internet 200, the processing to one client will be described for the sake of convenience.

First, the client 301 connected with the dial-up server 30 by a dial-up connection through the telephone network 100 or the client 401 connected with the router 60 through the Internet 200 commences the communication with the streaming server 40 (step S41). Then, a screen G1 is shown on the display unit of the client 301 or 401 to prompt for input of preinformed authentication data (ID and password) from the client 301 or 401 to obtain distributed images and others, and the processing unit 41 receives those inputted authentication data therefrom (step S42).

When the inputted authentication data are invalid (step S43 = "NO"), the processing unit 41 notifies that the data are invalid (step S44), and the unit 41 terminates the processing. When the inputted authentication data are valid (step S43 = "YES"), the processing proceeds to a step S45. In the step S45, the file type previously set up and stored in the distribution setting memory unit 51 is read out. When a multi-rate encoding is set (step S45 = "YES"), a screen G2 is shown to prompt for input selecting an encoding rate and an image source (step S46).

The screen G2 indicates that the encoding rates are set at 50 kbps and 100 kbps, and that two of the video cameras 71 and 72 (they correspond to a button for camera 1 and a button for a camera 2 on the screen G2.) and one image pickup device 92 attached to the surgical microscope (medical optical device) 90 are connected with the encoding server 20. The display on the screen G2 includes check buttons G2a and G2b for selecting encoding rates, check buttons G2c, G2d, and G2e for selecting an image source, and an entry button G2f for transmitting selected details to the distribution apparatus 10. Then, the recipient of image distribution clicks on check buttons (for example, G2a and G2e) for an encoding rate, which is appropriate for the communication environment of the client 301 or 401, and for images to observe, and then he/she clicks on the entry button G2f to enter the selected details.

On the other hand, when the single rate encoding is selected (step S45 = "NO"), a screen G3 is shown to prompt for input selecting an image source (step S47). The screen G3 indicates that two video cameras 71 and 72 and one image pickup device 92 attached to the surgical microscope (medical optical device) 90 are connected with the encoding server 20. The display on the screen G3 includes check buttons G3a, G3b, and G3c for selecting an image source and an entry button G3d for transmitting selected details to the distribution apparatus 10. Then, the recipient of image distribution clicks on a check button (for example, G3c) for the image he/she desires to observe and clicks on the entry button G3d to enter the selected image source.

A step S48 will be described under two conditions where the single rate encoding is set for the file type of the distribution setting (condition 1), and where the multi-rate encoding is set for the file type of the distribution setting (condition 2). Under the condition 1, the processing unit 41 sends the image streaming signals at the encoding rate set for the selected image to the client 301 or 401 because the streaming server 40 receives only the image streaming signals at the encoding rate set for the distribution setting. Under the condition 2, the image streaming signals at plural encoding rates set for the distribution setting are given regarding the video cameras 71, 72, ... and the image pickup device 92, but the processing unit 41 starts to send only the image streaming signals at the selected encoding rate for the selected image to the client 301 or 401 in real time. In both the conditions (condition 1 and condition 2), the processing unit 41 starts to send a voice bit stream given from the encoding server 20, as well as the image streaming signals, to the client 301 or 401 in this step.

Next, in a step S49, the patient data to be distributed set by the patient data distribution setup processing are read out from the patient data distribution memory unit 53 for distribution. In a step S50, the processing unit 41 sends vital sign data given in sequence from the encoding server 20 to the client 301 or 401 in real time. The client 301 or 401 has a WWW browser and an image streaming replay program, whereby the information distributed in the steps S48, S49, and S50 is received by the client 301 or 401 to be displayed on the display unit thereof. Further, in a step S51, the processing unit 41 shows an image changing button G4d on the display unit of the client 301 or 401.

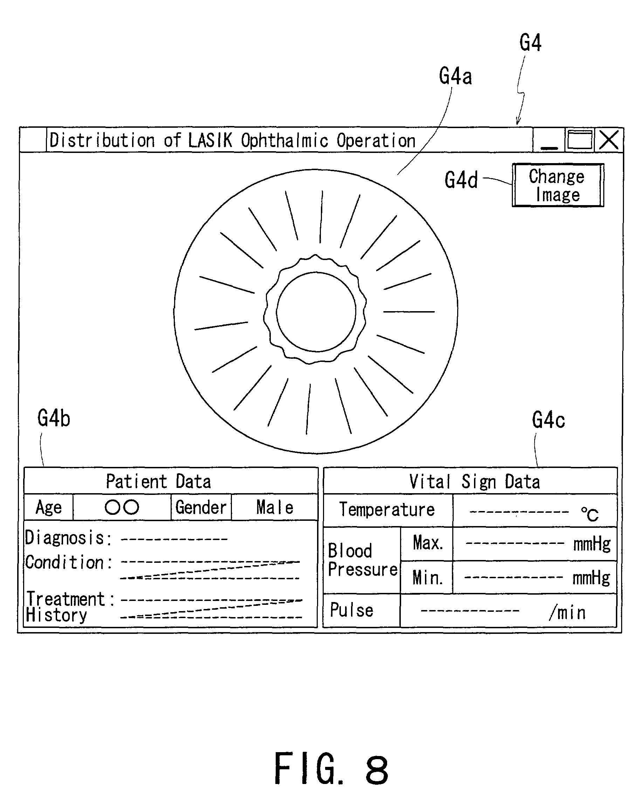

Fig. 8 is view showing a layout of a screen displayed on the display unit of the client 301 or 401 at the time of receiving image streaming signals from the streaming server 40. A screen G4 includes display areas for displaying distributed data: a display area G4a is for displaying moving images that the distributed streaming signals represent, a display area G4b for patient data, and a display area G4c for vital sign data. Further, an image changing button G4d appears on the display unit of the client 301 or 401. For example, when the surgical microscope (medical optical device) 90 is selected as an image source in the steps S46 and S47, the moving images picked up by the microscope (medical optical device) 90 are displayed in the display area G4a in real time.

When the image changing button G4d is clicked (step S52 = "YES" , condition 1 ) , a signal for demanding an image change is given from the client 301 or 401 to the streaming server 40. In response to the signal, the processing unit 41 brings the processing back to the step 45 and then carries out the steps from S45 to S51 again to switch the current moving images displayed on the display area G4a to other moving images. For example, when the camera 1 is selected in the step S46 or S47, images displayed in the display area G4a are the moving images taken by the video camera 71 (camera 1) disposed to pick up the moving images of the entire operating room. From then on, the images to be displayed on the display area G4a may be changed at the client's end in the same way.

As described above, the image distribution apparatus 10 distributes moving images in real time to the clients 301, 302, 303, ... connected through the telephone network 100 as well as to the clients 401, 402, 403, ... connected through the Internet 200. Moreover, it is possible, at the client's end, to select the image to observe from more than one image, to change the aiming directions of an image pickup, and to adjust zooming scales as well.

Further, when the remote control computer 500 is connected with the image distribution apparatus 10 through the telephone network 100, the processing unit 41 carries out the remote control program 48, whereby the remote control computer 500 is capable of remotely controlling distribution setting regarding the encoding rate and others, patient data distribution setting and others involving distribution, aiming directions of an image pickup and a zooming operation, switching among two or more cameras, maintenance in case of trouble, and an update of software, and so on.

According to the preferred embodiment described above, only one image pickup means is selected in the steps S46 and S47, but it may be possible to select more than one image pickup means. When more than one image pickup means is selected, plural moving images are simultaneously shown on the display area G4a of the client 301 or 401. Furthermore, selection of images to be distributed does not need to be dependent on recipients' image selection. In other words, by skipping the processing of the image selection in the steps S46 and S47, the images picked up by all the image pickup means connected with the image distribution apparatus 10 may be distributed, and plural moving images may be shown on the display area of the client 301 or 401 simultaneously.

In addition, the vital sign data measured by the vital sign measurement device 85 and distributed to the client 301 or 401 may not be limited to a combination of the data concerning temperature, blood pressure, and pulses, which are described as examples in the preferred embodiment. The vital data may be a part of those data (for example, only the data about pulses), and the vital data may include other measured values such as oxygen saturation in the blood, a number of breaths, and so on.

The present invention may not be limited to the preferred embodiments described above, and it may be altered in many respects without deviating from the principles of the present invention. For example, only one image pickup device 92 attached to the surgical optical device 90 is connected with the image distribution apparatus 10, but more than one image pickup device and/or more than one microphone may be connected with the apparatus 10.

The foregoing description of the preferred embodiments of the invention has been presented for purposes of illustration and description. It is not intended to be exhaustive or to limit the invention to the precise form disclosed, and modifications and variations are possible in the light of the above teachings or may be acquired from practice of the invention. The embodiments chosen and described in order to explain the principles of the invention and its practical application to enable one skilled in the art to utilize the invention in various embodiments and with various modifications as are suited to the particular use contemplated. It is intended that the scope of the invention be defined by the claims appended hereto, and their equivalents.

## Claims

1. An image distribution apparatus comprising:
dial-up communication means for carrying out digital communication with one or more clients through a telephone network;
Internet communication means for carrying out digital communication with one or more clients through the Internet;
interface means for at least receiving a moving image signal picked up by image pickup means attached to a medical device;
image encoding means for encoding the moving image signal to a digitized image signal at one or more preset data rates in real time; and
image distribution means for distributing at least one digitized image signal at one or more of the data rates to the one or more clients through the telephone network or the Internet.

2. The image distribution apparatus according to claim 1, wherein the medical device includes a surgical microscope.

3. The image distribution apparatus according to claim 1, wherein the medical device includes a slit lamp.

4. The image distribution apparatus according to any one of claims 1 to 3, further comprising:
voice input means for inputting a voice signal;
voice grade selecting means for selecting a voice grade for encoding;
voice encoding means for encoding the voice signal inputted by the voice input means to a digitized voice signal at a data rate conforming to the voice grade selected by the voice grade selecting means; and
voice distribution means for distributing the digitized voice signal to the one or more clients through the telephone network or the Internet.

5. The image distribution apparatus according to any one of claims 1 to 4,
wherein the interface means receives plural moving image signals,
the image encoding means encodes each of the plural moving image signals to a digitized image signal, and
the image distribution means distributes all or a part of the plural digitized image signals to the one or more clients.

6. The image distribution apparatus according to claim 5, wherein the image distribution means distributes only an image signal selected from among the plural image signals in accordance with an image selecting signal from the client.

7. The image distribution apparatus according to any one of claims 1 to 6, further comprising:
data rate memory means for prestoring plural sets of data rate groups consisted of one or more of the data rates; and
data rate setting means for setting the data rates of the digitized image signal by selecting from the plural stored sets of the data rate groups; and
wherein the image encoding means encodes the image signal to the digitized image signal at all the rates included in the selected data rate group.

8. The image distribution apparatus according to any one of claims 1 to 7, further comprising:
interface means for receiving a measurement signal indicating a value of a vital sign including at least one selected from temperature, blood pressure, pulses, the number of breaths, and oxygen saturation in blood;
measurement signal encoding means for encoding the measurement signal to a digitized measurement signal in real time; and
measurement signal distribution means for distributing the digitized measurement signal to the one or more clients through the telephone network or the Internet.

9. The image distribution apparatus according to any one of claims 1 to 8, further comprising:
patient identifying data input means, connected with a patient information database, for inputting patent identifying data identifying a patient to be picked up by the image pickup means; and
data distribution means for searching patient data corresponding to the patient identifying data through the patient information database when the patient identifying data are inputted by the patient identifying data input means, and for distributing all or a part of the patient data to the one or more clients through the telephone network or the Internet.
